Europäisches Patentamt

(19) European Patent Office-

Office européen des brevets

(11) Publication number: **0 108 849**
**B1**

(12) **EUROPEAN PATENT SPECIFICATION**

(45) Date of publication of patent specification: **06.05.87**

(21) Application number: **82306100.7**

(22) Date of filing: **16.11.82**

(51) Int. Cl.⁴: **G 05 D 11/13,** G 01 N 29/00,
G 01 F 1/74

(54) **Apparatus and method for controlling consistency.**

(43) Date of publication of application:
**23.05.84 Bulletin 84/21**

(45) Publication of the grant of the patent:
**06.05.87 Bulletin 87/19**

(84) Designated Contracting States:
**DE FR GB IT SE**

(56) References cited:
**DE-B-1 296 420**
**FR-A-1 197 130**
**GB-A-2 040 453**

㎄ Proprietor: **GENERAL SIGNAL CORPORATION**
**PO Box 10010 High Ridge Park**
**Stamford Connecticut 06904 (US)**

(72) Inventor: **Greey, David Norman**
**67 Teddington Park Avenue**
**Toronto Ontario M4N 2C5 (CA)**

(74) Representative: **Baillie, Iain Cameron et al**
**c/o Ladas & Parry Isartorplatz 5**
**D-8000 München 2 (DE)**

Courier Press, Leamington Spa, England.

## Description

The present invention relates to the control of consistency of slurries, particularly paper stock used in paper making processes, and also to an improved method and apparatus for measuring the consistency of such slurries.

The invention is especially suitable for the control of consistency in a paper pulp or fiber stock tower which is a tank containing high density stock. The invention may also be applied for the measurement and control of consistency of other applications in paper making, as for example in blow tanks, stock chests, broke tanks, bleach plant towers as are used for chlorine retention, caustics extraction and hypochlorination. Other applications for the invention may be found wherever the consistency of materials which may be stored in slurry form requires measurement or control.

Consistency is a measure of the percent by weight of solids in a liquid slurry of such solids. It is a term well known and used in the pulp and paper making industry. Various devices have been proposed and are commercially available for the measurement and control of consistency of paper stock. Typically, consistency is measured in a pipe or other channel carrying the outflow of the paper stock from a tank. The dilution of the stock in the tank is controlled by controlling the supply of make-up water to the tank. In order to control consistency in this manner, the incoming stock must be higher in consistency than is desired to permit the controller to operate. Inasmuch as the measurement is made after the dilution process, the measurement does not accurately reflect the actual consistency of the material in the tank. Further dilution or trimming downstream from the tank and the measurement point may be required to obtain the consistency specified for the further processing of the material.

It has been discovered that accurate measurement and control of consistency can be obtained directly in the vessel or tank containing the slurry by utilizing the flow produced by the agitator or mixing impeller or pulper which circulates the slurry within a region of the vessel. The flow is preferably detected by a sonic flow sensor which responds to the velocity of the particles of material while the flow thereof is induced, by shift in frequency between transmitted and received waves due to the Doppler effect. This velocity signal obtained from the sensor is used to vary the dilution of the slurry thereby controlling the consistency thereof. The addition of make-up material, such as dilutant, the outlet slurry of controlled consistency, as well as the measurement of consistency are all made in the same region of the tank where flow is occurring. The measurement and control of consistency is closely coupled and is carried out without delay thereby resulting in maximum accuracy. Downstream dilution and trimming to obtain desired consistency can be eliminated in that lower consistencies of the material in the tank result in a reduction of size in the motor and drive for operating the agitator with the attendant conservation and saving of power.

Accordingly, it is principal object of the present invention to provide an improved method and apparatus for the measurement and control of consistency in slurries which may be contained in a chest or tank.

It is a further object of the present invention to provide an improved method and apparatus for the measurement and control of consistency at low cost and with high accuracy.

According to the invention, these objects are achieved by the apparatus according to Claim 1 and the method of Claim 8.

The foregoing objects, features and advantages of the invention, as well as the best mode known for practicing the invention and a presently preferred embodiment thereof, will become more apparent from a reading of the following description in connection with the accompanying drawings in which:

FIG. 1 is a schematic diagram of consistency control apparatus in accordance with the invention showing the paper stock tower in which the components of the invention are installed in elevation;

FIG. 2 is a sectional plan view, taken along the line 2—2 in FIG. 1 so as to illustrate the location of the sensor and impeller components shown in FIG. 1.

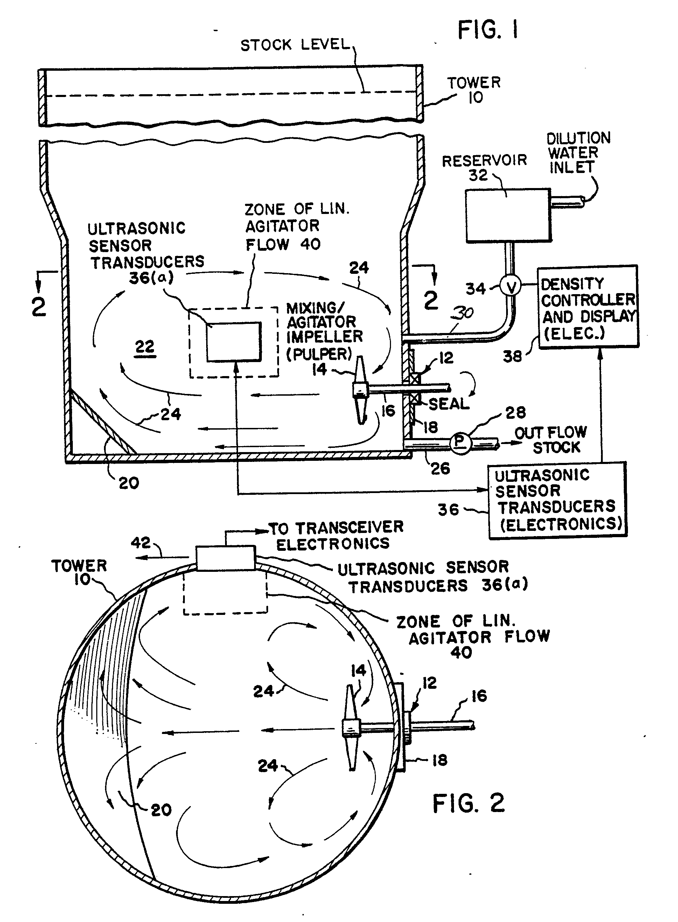

Referring to FIG. 1 there is shown a tank 10 known as a stock tower which contains a slurry of pulp and/or fiber in water. Near the bottom of the tower 10 there is located a side entering agitator or mixer unit 12. The unit 12 has an impeller 14 or pulper at the end of a shaft 16. The shaft is driven by a motor drive (not shown), a flange and seal arrangement 18 holds the unit on the outside of the tower 10. The tower is cylindrical and has a vertical axis. It may, however, be rectilinear in horizontal cross section. A fillet 20 in the lower corner of the tank opposite to the impeller 14 aids the flow of the slurry induced by the impeller in a region 22 at the bottom of the tank. The arrows 24 generally indicate the direction in which the slurry circulates around the region 22 as it flows.

An outlet pipe 26 is coupled to the bottom of the tower 10 below the agitator 12. A pump 28 may be used to obtain an outlet flow of the stock through the pipe 26. It will be noted that the outlet is in the region 22 where flow is induced by the agitator 12.

Inlet of make-up material, illustrated as dilution water, is also into the region 22 where flow is induced by the agitator 12. An inlet pipe 30 enters the region above the impeller and on the opposite side of the impeller from the outlet pipe 26. The dilutant water may be obtained from a reservoir 32 or other source of low pressure water which is connected to the pipe 30. A throttle valve 34 in the pipe controls the flow of the dilution water, and therefore the amount of water which is added in order to obtain the desired consistency of the stock which flows through the outlet pipe 26.

A flow sensor 36 which detects the velocity or rate of movement (flow) of the slurry provides an output from which a measurement of consistency in the region 22 is desired. This sensor is preferably a sonic sensor which obtains a velocity measurement by the Doppler shift effect. The term sonic as used herein includes acoustic waves both audible and inaudible. The frequency of the waves depends upon the nature of the material upon the dimensions of the region 22. It has been found that a commercial ultrasonic sensor having a transcevier and sensor transducers 36a may be used. The transducer projects ultrasonic waves into the circulating slurry in the region 22. These waves are reflected from the particulate portion of the slurry and are frequency shifted, on average, in accordance with the average velocity of the particulate component of the slurry. This velocity measurement is obtained by an FM discriminator in the transceiver and provides an output signal to a display and density controller unit 38 on which a measurement of the consistency is obtained.

The output is used to control the valve 34, which may be an electromechanical servo valve, so as to increase and decrease the flow of dilution water, as measured velocity decreases and increases, so as to maintain the consistency of the slurry as it flows from the outlet pipe 22 at a predetermined consistency.

It has been found that the accuracy of the consistency measurement and the accuracy of consistency control is enhanced when the sensor transducers 36a are located in a zone 40 where the flow is linear. It has been found that this flow is linear in the inner wall surface where a tangent line 42 parallel to the access of the shaft 16 of the agitator 12 intersects the wall surface at a height above the horizontal plane of the agitator shaft 16 which depends upon the nature of the slurry and the diameter of the tower. In order to present the sensor transducers 36a to the slurry in the zone of linear agitator flow 40 a plate in which the transducers are mounted may be installed in place of a section of the wall of the tower 10 as shown in FIG. 2. The zone of linear flow (i.e. wherein the slurry is moving at linear velocity) may be at another location than along the inside wall of the tank as shown in FIGS. 1 and 2, when the tank cross-section is not circular. In such cases the transducers may be mounted on a support so as to extend from the wall or bottom of the tank into the zone. Such mounting is not preferred in that it may interfere with the flow pattern and may have to be maintained to clear stock which is deposited thereon. Various pulp or fiber chest may be used other than the illustrated stock tower 10.

## Claims

1. Apparatus for monitoring the consistency of a slurry contained in a vessel (10) comprising means (12, 14, 16) for generating a circulating flow of said slurry in a region (22) of said vessel, the apparatus being characterised by means (36, 36a) for sensing velocity of said flow to derive a signal representative of the consistency of the slurry.

2. Apparatus as claimed in claim 1 wherein said means for sensing the velocity of the flow of slurry comprises a Doppler effect device (36, 36a) responsive to the frequency shift betwen sonic signals transmitted into and received from said flow to derive a signal representative of the flow velocity.

3. Apparatus as claimed in claim 1 or 2 wherein said circulating flow of slurry in a region (22) of the vessel (10) is arranged so as to be linear in a zone (40) of said region, and the flow velocity of the slurry is sensed in said zone.

4. Apparatus as claimed in claim 3 wherein said vessel comprises a cylindrical tank (10) having its axis vertical and said flow generating means comprises a rotary impeller (14) having a horizontal shaft (16) extending radially with respect to the tank axis, and wherein said zone (40) is located adjacent the cylindrical wall of said tank at a predetermined vertical height above the impeller shaft and radially of the tank axis in a direction generally transverse to the impeller shaft.

5. Apparatus as claimed in any preceding claim and further comprising means (34, 38) responsive to the sensed velocity of the circulating flow of slurry for controlling dilution of the slurry thereby to control the consistency thereof.

6. Apparatus as claimed in claim 5 wherein an inlet is provided into said vessel (10) for admitting diluting medium into the region (22) of the vessel wherein said flow generating means (12, 14, 16) is operative and means (34) are provided for determining the flow of diluting medium into said inlet, means (38) beng provided for controlling the operation of the last-mentioned means in dependance upon the sensed slurry flow velocity.

7. Apparatus as claimed in claim 6 wherein said means for determining the flow of diluting medium comprises a valve (34) in a pressurised flow line (30) to said inlet and said control means (38) operates said valve to throttle variably said pressurized flow line.

8. A method for the measurement of the consistency of a slurry in a tank (10) which comprises the step of agitating said slurry with an impeller (14) to produce a circulating flow of said slurry in a region (22) of said tank, the method being characterized by the steps of detecting the velocity at which said slurry flows in said region, and deriving a measurement of consistency from said velocity.

## Patentansprüche

1. Vorrichtung zum Überwachen der Konsistenz einer in einem Gefäß (10) enthaltenen Aufschlämmung mit Mitteln (12, 14, 16) zum Erzeugen einer Umlaufströmung der Aufschlämmung in einem Bereich (22) des Gefäßes, wobei die Vorrichtung gekennzeichnet ist durch eine Einrichtung (36, 36a) zum Messen der Geschwindigkeit der genannten Strömung und zum Erzeugen eines davon abgeleiteten Signals, das die Konsistenz der Aufschlämmung darstellt.

2. Vorrichtung nach Anspruch 1, dadurch gekennzeichnet, daß die Einrichtung zum Messen der Strömungsgeschwindigkeit der Aufschlämmung eine Dopplereffekteinrichtung (36, 36a) umfaßt, die auf die Frequenzverschiebung zwischen in die Strömung eingeleiteten und aus ihr empfangenen akustischen Signalen durch Erzeugung eines Signals anspricht, das die Strömungsgeschwindigkeit darstellt.

3. Vorrichtung nach Anspruch 1 oder 2, dadurch gekennzeichnet, daß die Umlaufströmung der Aufschlämmung in einem Bereich (22) des Gefäßes derart erzeugt wird, daß die Strömung in einer Zone (40) des genannten Bereiches linear ist, und daß die Strömungsgeschwindigkeit der Aufschlämmung in der genannten Zone gemessen wird.

4. Vorrichtung nach Anspruch 3, dadurch gekennzeichnet, daß das Gefäß einen zylindrischen Behälter (10) mit vertikaler Achse besitzt, daß die strömungserzeugende Einrichtung ein rotierendes Flügelrad (14) mit einer horizontalen Welle (16) besitzt, die sich in Bezug auf die Behälterachse radial erstreckt, und daß die genannte Zone (40) in der Nähe der zylindrischen Wand des Behälters in einer vorherbestimmten vertikalen Höhe oberhalb der Flügelradwelle und in einer allgemein quer zu der Flügelradwelle verlaufenden Richtung im Radialabstand von der Flügelradwelle angeordnet ist.

5. Vorrichtung nach einem der vorhergehenden Ansprüche, mit einer Einrichtung (34, 38), die auf die gemessene Geschwindigkeit der Umlaufströmung der Aufschlämmung durch eine Steuerung der Verdünnung der Aufschlämmung zwecks Steuerung ihrer Konsistenz anspricht.

6. Vorrichtung nach Anspruch 5, dadurch gekennzeichnet, daß das Gefäß (10) mit einem Einlaß zum Einleiten eines Verdünnungsmediums in jenen Bereich (22) des Gefäßes versehen ist, in dem die strömungserzeugende Einrichtung (12, 14, 16) arbeitet, daß eine Einrichtung (34) vorgesehen ist, die den Zufluß des Verdünnungsmediums in den Einlaß bestimmt, und daß eine Einrichtung (38) zur Steuerung des Betriebes der zuletztgenannten Einrichtung in Abhängigkeit von der gemessenen Strömungsgeschwindigkeit der Aufschlämmung vorgesehen ist.

7. Vorrichtung nach Anspruch 6, dadurch gekennzeichnet, daß die Einrichtung zum Bestimmen des Zuflusses des Verdünnungsmediums ein Ventil (34) in einer zu dem Einlaß führenden Druckstromleitung (30) umfaßt und daß die Steuereinrichtung (38) zwecks veränderbarer Drosselung der Druckstromleitung das Ventil betätigt.

8. Verfahren zum Messen der Konsistenz einer Aufschlämmung in einem Behälter (10) mit folgenden Schritten: durch Rühren der Aufschlämmung mit einem Flügelrad (14) wird in einem Bereich (22) des Behälters eine Umlaufströmung der Aufschlämmung erzeugt, wobei das Verfahren dadurch gekennzeichnet ist, daß die Strömungsgeschwindigkeit der Aufschlämmung in dem genannten Bereich gemessen und von dieser Geschwindigkeit ein Maß der Konsistenz abgeleitet wird.

## Revendications

1. Appareil pour surveiller la consistance d'une suspension contenue dans une enceinte (10) comprenant des moyens (12, 14, 16) pour produire un écoulement en circulation de ladite suspension dans une région (22) de la chambre, cet appareil étant caractérisé par des moyens (36, 36a) pour capter la vitesse de l'écoulement et pour fournir un signal représentatif de la consistance de la suspension.

2. Appareil selon la revendication 1, dans lequel les moyens pour capter la vitesse d'écoulement de la suspension comprennent un dispositif à effet Doppler (36, 36a) sensible au décalage de fréquence entre des signaux sonoriques émis et reçus à partir dudit écoulement pour fournir un signal représentatif de la vitesse de l'écoulement.

3. Appareil selon l'une des revendications 1 ou 2, dans lequel l'écoulement en circulation de la suspension dans une région (22) de la chambre (10) est conçu pour être linéaire dans une zone (40) de cette région et la vitesse d'écoulement de la suspension est détectée dans cette zone.

4. Appareil selon la revendication 3, dans lequel la chambre comprend un réservoir cylindrique (10) dont l'axe est vertical et dans lequel le moyen de génération d'écoulement comprend un impulseur tournant (14) ayant un axe horizontal (16) s'étendant radialement par rapport à l'axe du réservoir, et dans lequel la zone (40) est située de façon adjacente à la paroi cylindrique du réservoir à une hauteur verticale prédéterminée au-dessus de l'arbre de l'impulseur et radialement par rapport à l'axe du réservoir dans une direction généralement transversal à l'arbre de l'impulseur.

5. Appareil selon l'une quelconque des revendications précédentes, comprenant en outre des moyens (34, 38) sensibles à la vitesse captée de l'écoulement en circulation de la suspension pour commander la dilution de la suspension et commander ainsi sa consistance.

6. Appareil selon la revendication 5, dans lequel une entrée est prévue dans la chambre (10) pour admettre un agent de dilution dans la région (22) de la chambre dans laquelle le moyen de génération d'écoulement (12, 14, 16) agit et dans lequel des moyens (34) sont prévus pour déterminer le débit de l'agent de dilution dans ladite entrée, des moyens (38) étant prévus pour commander le fonctionnement du dernier moyen mentionné en relation avec la vitesse captée de l'écoulement de suspension.

7. Appareil selon la revendication 6, dans lequel les moyens pour déterminer le débit de l'agent de dilution comprennent une valve (34) dans une conduite d'écoulement sous pression (30) vers ladite entrée et dans lequel moyens de commande (38) actionnent ladite valve pour étrangler de façon variable la conduite d'écoulement sous pression.

8. Procédé de mesure de la consistance d'une suspension dans un réservoir (10) qui comprend l'étape consistant à agiter la suspension à l'aide d'un impulseur (14) pour fournir un écoulement en circulation de ladite suspension dans une région (22) du réservoir, ce procédé étant caractérisé par les étapes consistant à capter la vitesse à laquelle la suspension s'écoule dans ladite région et à déduire une mesure de consistance à partir de cette vitesse.

FIG. 1

FIG. 2